# EUROPEAN PATENT SPECIFICATION

(11) **EP 2 712 311 B1**
(45) Date of publication and mention of the grant of the patent: **12.06.2019**
(21) Application number: 12774641.0
(22) Date of filing: 03.04.2012
(51) Int. Cl.: A61F 9/01, A61F 9/008, A61N 5/06, A61F 9/007, A61B 18/00

(54) **CONTROLLED CROSS-LINKING INITIATION AND CORNEAL TOPOGRAPHY FEEDBACK SYSTEMS FOR DIRECTING CROSS-LINKING**
GESTEUERTE VERNETZUNGSINITIIERUNG UND HORNHAUTTOPOGRAFIE-FEEDBACKSYSTEME FÜR DIREKTE VERNETZUNG
DÉBUT DE RÉTICULATION CONTRÔLÉE ET SYSTÈME DE RÉTROACTION TOPOGRAPHIQUE CORNÉENNE

(30) Priority: 20.04.2011 US 201161477505 P; 08.08.2011 US 201161521261 P; 02.10.2011 US 201161542269 P; 24.10.2011 US 201161550576 P
(43) Date of publication of application: 02.04.2014
(73) Proprietor: Avedro, Inc., Waltham, MA 02451 (US)
(72) Inventor: MULLER, David, Boston, MA 02111 (US); MARSHALL, John, Farnborough Hampshire GU14 7AU (GB); FRIEDMAN, Marc, Needham, Massachusetts 02494 (US); BLINN, Stephen, Amherst, New Hampshire 03031 (US); SCHARF, Stephen, Waltham, Massachusetts 02451 (US); KAMAEV, Pavel, Lexington, Massachusetts 02421 (US); PERTAUB, Radha, Watertown, Massachusetts 02472 (US)
(74) Representative: Mewburn Ellis LLP
(86) International application number: PCT/US2012/032024
(87) International publication number: WO 2012/145159

(56) References cited:
- WO-A1-2007/139927
- WO-A2-2011/116306
- US-A1- 2009 149 842
- US-A1- 2009 149 923
- US-A1- 2009 171 305
- US-A1- 2009 171 305
- US-A1- 2009 275 929
- US-A1- 2010 318 017

## Description

### BACKGROUND OF THE INVENTION

### Field of the Invention

The invention pertains to systems and methods for stabilizing corneal tissue, and more particularly, systems and methods for applying and activating a cross-linking agent in corneal tissue and monitoring the activation of the cross-linking agent.

### Description of Related Art

A variety of eye disorders, such as myopia, keratoconus, and hyperopia, involve abnormal shaping of the cornea. Laser-assisted in-situ keratomileusis (LASIK) is one of a number of corrective procedures that reshape the cornea so that light traveling through the cornea is properly focused onto the retina located in the back of the eye. During LASIK eye surgery, an instrument called a microkeratome is used to cut a thin flap in the cornea. The cornea is then peeled back and the underlying cornea tissue ablated to the desired shape with an excimer laser. After the desired reshaping of the cornea is achieved, the cornea flap is put back in place and the surgery is complete.

In another corrective procedure that reshapes the cornea, thermokeratoplasty provides a noninvasive procedure that applies electrical energy in the microwave or radio frequency (RF) band to the cornea. In particular, the electrical energy raises the corneal temperature until the collagen fibers in the cornea shrink at about 60°C. The onset of shrinkage is rapid, and stresses resulting from this shrinkage reshape the corneal surface. Thus, application of energy according to particular patterns, including, but not limited to, circular or annular patterns, may cause aspects of the cornea to flatten and improve vision in the eye.

The success of procedures, such as LASIK or thermokeratoplasty, in addressing eye disorders, such as myopia, keratoconus, and hyperopia, depends on the stability of the changes in the corneal structure after the procedures have been applied.

### BRIEF SUMMARY

The present invention is described and its scope determined by the appended claims. Aspects of the present disclosure further provide a system for applying a controlled amount of cross-linking in corneal tissue of an eye. Such systems are known for example from WO 2011/116306. The system includes an applicator adapted to apply a cross-linking agent to the eye. The system also includes a light source adapted to emit a photoactivating light. The system also includes a targeting system adapted to create targeting feedback information indicative of a position of a cornea of the eye. The system also includes a mirror array having a plurality of mirrors arranged in rows and columns. The plurality of mirrors are adapted to selectively direct the photoactivating light toward the eye according to a pixelated intensity pattern having pixels corresponding to the plurality of mirrors in the mirror array. The system also includes an interferometer adapted to monitor an amount of cross-linking in the corneal tissue. The interferometer monitors the amount of cross-linking in the corneal tissue by interfering a beam of light reflected from a surface of the eye with a reference beam of light reflected from a reference surface. The interferometer monitors the amount of cross-linking in the corneal tissue by also capturing, via an associated camera, a series of images of interference patterns due to optical interference between the beam of light and the reference beam of light. The series of images are indicative of a plurality of profiles of the surface of the eye. The system also includes a head restraint device for restraining a position of a head associated with the eye. The head restraint device thereby aligns the eye with respect to the interferometer. The system also includes a controller. The controller is adapted to receive the targeting feedback information and receive the generated series of intensity patterns. The controller is also adapted to analyze the generated series of intensity patterns to determine the plurality of profiles of the surface of the eye associated therewith. The controller is also adapted to determine an amount of cross-linking of the corneal tissue based on a dynamic deformation of the surface of the eye. The dynamic deformation of the eye is indicated by the plurality of profiles of the surface of the eye. The controller is also adapted to adjust the pixelated intensity pattern according to data. The data includes at least one of: the targeting feedback information and the determined amount of cross-linking.

These and other aspects of the present disclosure will become more apparent from the following detailed description of embodiments of the present disclosure when viewed in conjunction with the accompanying drawings.

### BRIEF DESCRIPTION OF THE DRAWINGS

FIG. 1 provides a block diagram of an example delivery system for delivering a cross-linking agent and an activator to a cornea of an eye in order to initiate molecular cross-linking of corneal collagen within the cornea.
FIG. 2A provides a flowchart showing an example embodiment according to aspects of the present disclosure for activating cross-linking within cornea tissue using a cross-linking agent and an initiating element.
FIG. 2B provides a flowchart similar to FIG. 2A where Riboflavin may be applied topically as the cross-linking agent and UV light may be applied as the initiating element.
FIG. 2C provides a flowchart similar to FIG. 2A, but with an additional step for placing a mask on the eye described in FIGS. 10A and 10B.
FIG. 3 provides an example delivery system for delivering light to the cornea 2 employing laser scanning technology.
FIG. 4 illustrates a delivery system incorporating a feedback system.
FIG. 5A illustrates a delivery system for activating cross-linking in the cornea with the laser scanning device and having a video camera feedback system.
FIG. 5B illustrates an exemplary operation of the delivery system shown in FIG. 5A.
FIG. 6 illustrates an example delivery system for applying light to an eye from a laser light source.
FIG. 7A illustrates an optical power contour map of an eye prior to initiation of cross-linking therapy.
FIG. 7B illustrates an optical power contour map of the eye shown in FIG. 7A following treatment by cross-linking therapy according to an aspect of the present disclosure.
FIG. 7C illustrates a contour map of the difference between the contour map in FIG. 7B and the contour map shown in FIG. 7A.

### DETAILED DESCRIPTION

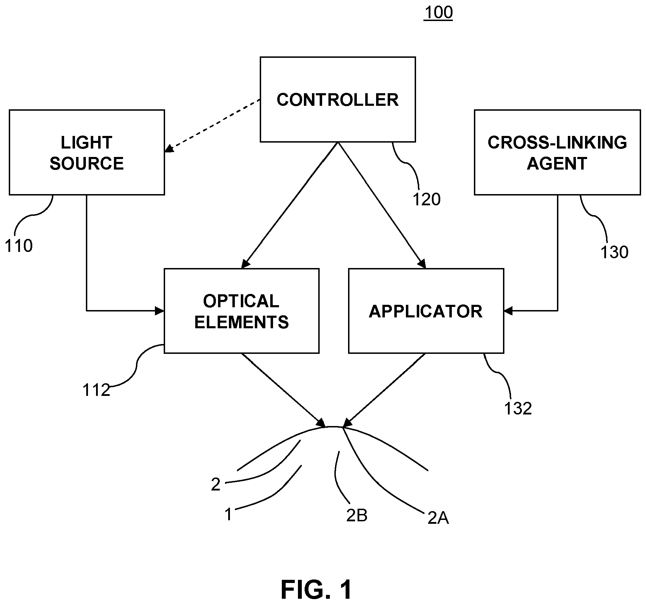

FIG. 1 provides a block diagram of an example delivery system 100 for delivering a cross-linking agent 130 and an activator to a cornea 2 of an eye 1 in order to initiate molecular cross-linking of corneal collagen within the cornea 2. Cross-linking can stabilize corneal tissue and improve its biomechanical strength. The delivery system 100 includes an applicator 132 for applying the cross-linking agent 130 to the cornea 2. The delivery system 100 includes a light source 110 and optical elements 112 for directing light to the cornea 2. The delivery system 100 also includes a controller 120 that is coupled to the applicator 132 and the optical elements 112. The applicator 132 may be an apparatus adapted to apply the cross-linking agent 130 according to particular patterns on the cornea 2 advantageous for causing cross-linking to take place within the corneal tissues. The applicator 132 may apply the cross-linking agent 130 to a corneal surface 2A *(e.g.,* an epithelium), or to other locations on the eye 1. Particularly, the applicator 132 may apply the cross-linking agent 130 to an abrasion or cut of the corneal surface 2A to facilitate the transport or penetration of the cross-linking agent through the cornea 2 to a mid-depth region 2B.

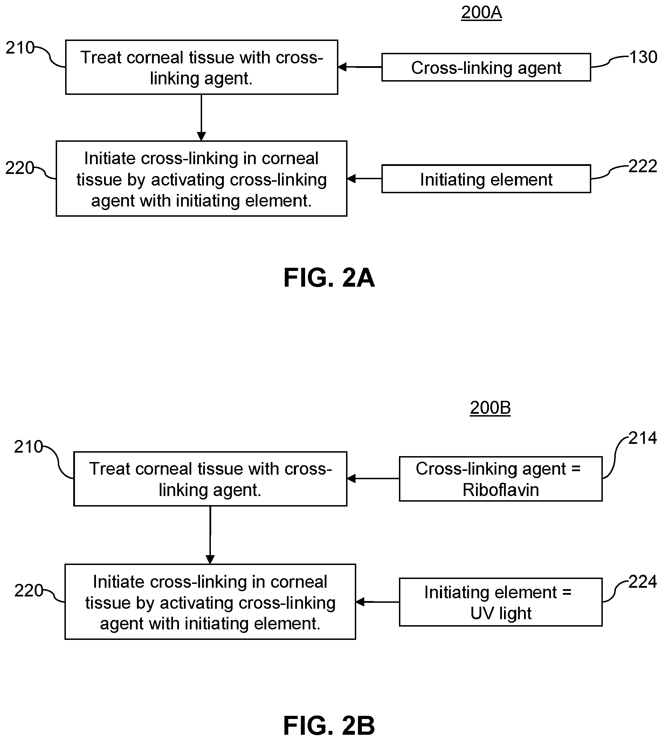

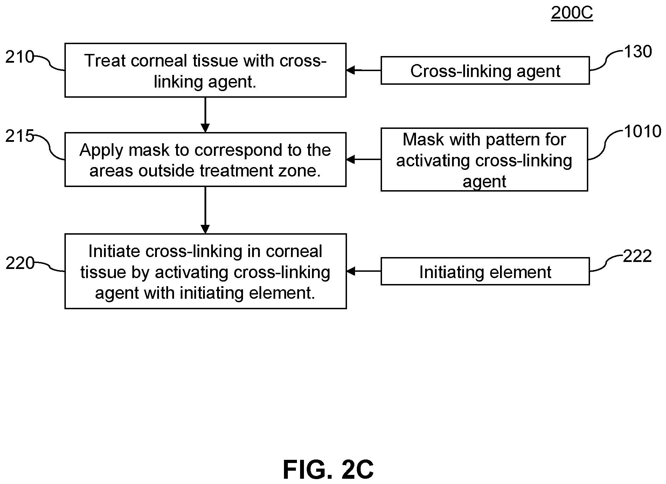

As described below in connection with FIGS. 2A-2B, which describe an exemplary operation of the delivery system 100, the cross-linking agent 130 is applied to the cornea 2 using the applicator 132. Once the cross-linking agent 130 has been applied to the cornea 2, the cross-linking agent 130 is initiated by the light source 110 (*i*.*e*. the initiating element) to cause cross-linking agent 130 to absorb enough energy to release free oxygen radicals within the cornea 2. Once released, the free oxygen radicals (*i*.*e*. singlet oxygen) form covalent bonds between corneal collagen fibrils and thereby cause the corneal collagen fibrils to cross-link and change the structure of the cornea 2. For example, activation of the cross-linking agent 130 with the light source 110 delivered to the cornea 2 through the optical elements 112 may result in cross-linking in the mid-depth region 2B of the cornea 2 and thereby strengthen and stiffen the structure of the cornea 2.

Although eye therapy treatments may initially achieve desired reshaping of the cornea 2, the desired effects of reshaping the cornea 2 may be mitigated or reversed at least partially if the collagen fibrils within the cornea 2 continue to change after the desired reshaping has been achieved. Indeed, complications may result from further changes to the cornea 2 after treatment. For example, a complication known as post-LASIK ectasia may occur due to the permanent thinning and weakening of the cornea 2 caused by LASIK surgery. In post-LASIK ectasia, the cornea 2 experiences progressive steepening (bulging).

Aspects of the present disclosure provide approaches for initiating molecular cross-linking of corneal collagen to stabilize corneal tissue and improve its biomechanical strength. For example, embodiments may provide devices and approaches for preserving the desired corneal structure and shape that result from an eye therapy treatment, such as LASIK surgery or thermokeratoplasty. In addition, aspects of the present disclosure may provide devices and approaches for monitoring the shape, molecular cross-linking, and biomechanical strength of the corneal tissue and providing feedback to a system for providing iterative initiations of cross-linking of the corneal collagen. As described herein, the devices and approaches disclosed herein may be used to preserve desired shape or structural changes following an eye therapy treatment by stabilizing the corneal tissue of the cornea 2. The devices and approaches disclosed herein may also be used to enhance the strength or biomechanical structural integrity of the corneal tissue apart from any eye therapy treatment.

Therefore, aspects of the present disclosure provide devices and approaches for preserving the desired corneal structure and shape that result from an eye treatment, such as LASIK surgery or thermokeratoplasty. In particular, embodiments may provide approaches for initiating molecular cross-linking of the corneal collagen to stabilize the corneal tissue and improve its biomechanical strength and stiffness after the desired shape change has been achieved. In addition, embodiments may provide devices and approaches for monitoring cross-linking in the corneal collagen and the resulting changes in biomechanical strength to provide a feedback to a system for inducing cross-linking in corneal tissue.

Some approaches initiate molecular cross-linking in a treatment zone of the cornea 2 where structural changes have been induced by, for example, LASIK surgery or thermokeratoplasty. However, it has been discovered that initiating cross-linking directly in this treatment zone may result in undesired haze formation. Accordingly, aspects of the present disclosure also provide alternative techniques for initiating cross-linking to minimize haze formation. In particular, the structural changes in the cornea 2 are stabilized by initiating cross-linking in selected areas of corneal collagen outside of the treatment zone. This cross-linking strengthens corneal tissue neighboring the treatment zone to support and stabilize the actual structural changes within the treatment zone.

With reference to FIG. 1, the optical elements 112 may include one or more mirrors or lenses for directing and focusing the light emitted by the light source 110 to a particular pattern on the cornea 2 suitable for activating the cross-linking agent 130. The light source 110 may be an ultraviolet light source, and the light directed to the cornea 2 through the optical elements 112 may be an activator of the cross-linking agent 130. The light source 110 may also alternatively or additionally emit photons with greater or lesser energy levels than ultraviolet light photons. The delivery system 100 also includes a controller 120 for controlling the operation of the optical elements 112 or the applicator 132, or both. By controlling aspects of the operation of the optical elements 112 and the applicator 132, the controller 120 can control the regions of the cornea 2 that receive the cross-linking agent 130 and that are exposed to the light source 110. By controlling the regions of the cornea 2 that receive the cross-linking agent 130 and the light source 110, the controller 120 can control the particular regions of the cornea 2 that are strengthened and stabilized through cross-linking of the corneal collagen fibrils. In an implementation, the cross-linking agent 130 can be applied generally to the eye 1, without regard to a particular region of the cornea 2 requiring strengthening, but the light source 110 can be directed to a particular region of the cornea 2 requiring strengthening, and thereby control the region of the cornea 2 wherein cross-linking is initiated by controlling the regions of the cornea 2 that are exposed to the light source 110.

The optical elements 112 can be used to focus the light emitted by the light source 110 to a particular focal plane within the cornea 2, such as a focal plane that includes the mid-depth region 2B. In addition, according to particular embodiments, the optical elements 112 may include one or more beam splitters for dividing a beam of light emitted by the light source 110, and may include one or more heat sinks for absorbing light emitted by the light source 110. The optical elements 112 may further include filters for partially blocking wavelengths of light emitted by the light source 110 and for advantageously selecting particular wavelengths of light to be directed to the cornea 2 for activating the cross-linking agent 130. The controller 120 can also be adapted to control the light source 110 by, for example, toggling a power switch of the light source 110.

In an implementation, the controller 120 may include hardware and/or software elements, and may be a computer. The controller 120 may include a processor, a memory storage, a microcontroller, digital logic elements, software running on a computer processor, or any combination thereof. In an alternative implementation of the delivery system 100 shown in FIG. 1, the controller 120 may be replaced by two or more separate controllers or processors. For example, one controller may be used to control the operation of the applicator 132, and thereby control the precise rate and location of the application of the cross-linking agent 130 to the cornea 2. Another controller may be used to control the operation of the optical elements 112, and thereby control with precision the delivery of the light source 110 (*i.e.* the initiating element) to the cornea 2 by controlling any combination of: wavelength, bandwidth, intensity, power, location, depth of penetration, and duration of treatment. In addition, the function of the controller 120 can be partially or wholly replaced by a manual operation. For example, the applicator 132 can be manually operated to deliver the cross-linking agent 130 to the cornea 2 without the assistance of the controller 120. In addition, the controller 120 can operate the applicator 132 and the optical elements 112 according to inputs dynamically supplied by an operator of the delivery system 100 in real time, or can operate according to a pre-programmed sequence or routine.

Referring to FIG. 2A, an example embodiment 200A according to aspects of the present disclosure is illustrated. Specifically, in step 210, the corneal tissue is treated with the cross-linking agent 130. Step 210 may occur, for example, after a treatment is applied to generate structural changes in the cornea and produce a desired shape change. Alternatively, step 210 may occur, for example, after it has been determined that the corneal tissue requires stabilization or strengthening. The cross-linking agent 130 is then activated in step 220 with an initiating element 222. In an example configuration, the initiating element 222 may be the light source 110 shown in FIG. 1. Activation of the cross-linking agent 130, for example, may be triggered thermally by the application of microwaves or light.

As the example embodiment 200B of FIG. 2B shows further, Riboflavin may be applied topically as a cross-linking agent 214 to the corneal tissue in step 210. As also shown in FIG 2B, ultraviolet (UV) light may be applied as an initiating element 224 in step 220 to initiate cross-linking in the corneal areas treated with Riboflavin. Specifically, the UV light initiates cross-linking activity by causing the applied Riboflavin to release reactive oxygen radicals in the corneal tissue. In particular, the Riboflavin acts as a sensitizer to convert O₂ into singlet oxygen which causes cross-linking within the corneal tissue.

According to one approach, the Riboflavin may be applied topically to the corneal surface, and transepithelial delivery allows the Riboflavin to be applied to the corneal stroma. In general, the application of the cross-linking agent sufficiently introduces Riboflavin to mid-depth regions of the corneal tissue where stronger and more stable structure is desired.

Where the initiating element is UV light, the UV light may be generally applied to the corneal surface 2A (*e*.*g*. the epithelium) of the cornea 2 to activate cross-linking. However, regions of the cornea 2 requiring stabilization may extend from the corneal surface 2A to a mid-depth region 2B in the corneal stroma 2C. Generally applying UV light to the corneal surface 2A may not allow sufficient penetration of the UV light to activate necessary cross-linking at a mid-depth region of the cornea. Accordingly, embodiments according to aspects of the present disclosure provide a delivery system that accurately and precisely delivers UV light to the mid-depth region 2B where stronger and more stable corneal structure is required. In particular, treatment may generate desired changes in corneal structure at the mid-depth region 2B.

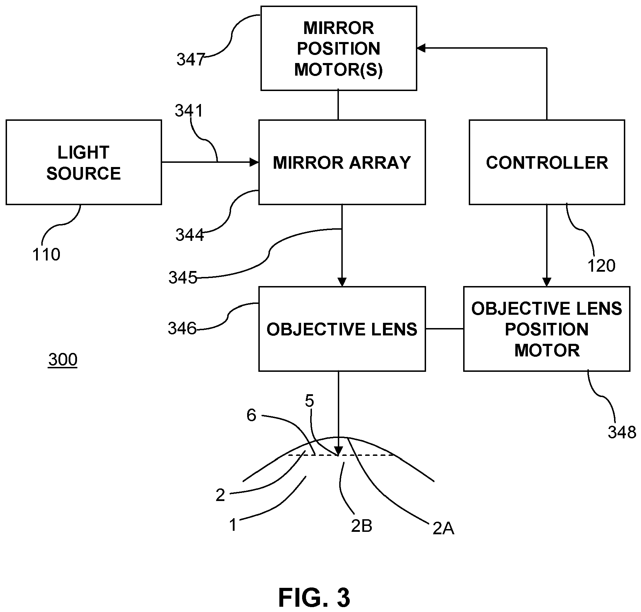

FIG. 3 provides an example delivery system adapted as a laser scanning device 300 for delivering light to the cornea 2 employing laser scanning technology. The laser scanning device 300 has the light source 110 for delivering a laser beam through an objective lens 346 into a small focal volume within the cornea 2. The laser scanning device 300 also includes the controller 120 for controlling the intensity profile of the light delivered to the cornea 2 using a mirror array 344 and for controlling the focal plane of the objective lens 346. The light source 110 can be an ultraviolet (UV) light source that emits a UV laser. A beam of light 341 is emitted from the light source 110 *(e.g.,* UV laser) and passes to the mirror array 344. Within the mirror array 344, the beam of light 341 from the light source 110 is scanned over multiple mirrors adapted in an array. The beam of light 341 can be scanned over the mirrors in the mirror array 344 using, for example, one or more adjustable mirrors to direct the beam of light 341 to point at each mirror in turn. The beam of light 341 can be scanned over each mirror one at a time. Alternately, the beam of light 341 can be split into one or more additional beams of light using, for example, a beam splitter, and the resultant multiple beams of light can then be simultaneously scanned over multiple mirrors in the mirror array 344.

By rapidly scanning the beam of light 341 over the mirrors in the mirror array 344, the mirror array 344 outputs a light pattern 345, which has a two dimensional intensity pattern. The two dimensional intensity pattern of the light pattern 345 is generated by the mirror array 344 according to, for example, the length of time that the beam of light 341 is scanned over each mirror in the mirror array 344. In particular, the light pattern 345 can be considered a pixilated intensity pattern with each pixel represented by a mirror in the mirror array 344 and the intensity of the light in each pixel of the light pattern 345 proportionate to the length of time the beam of light 341 scans over the mirror in the mirror array 344 corresponding to each pixel. In an implementation where the beam of light 341 scans over each mirror in the mirror array 344 in turn to create the light pattern 345, the light pattern 345 is properly considered a time-averaged light pattern, as the output of the light pattern 345 at any one particular instant in time may constitute light from as few as a single pixel in the pixelated light pattern 345. In an implementation, the laser scanning technology of the delivery system 300 may be similar to the technology utilized by Digital Light Processing™ (DLP®) display technologies.

The mirror array 344 can include an array of small oscillating mirrors, controlled by mirror position motors 347. The mirror position motors 347 can be servo motors for causing the mirrors in the mirror array 344 to rotate so as to alternately reflect the beam of light 341 from the light source 340 toward the cornea 2. The controller 120 can control the light pattern 345 generated in the mirror array 344 using the mirror position motors 347. In addition, the controller 120 can control the depth within the cornea 2 that the light pattern 345 is focused to by controlling the location of the focal depth of the objective lens 346 relative to the corneal surface 2A. The controller can utilize an objective lens position motor 348 to raise and/or lower the objective lens 346 in order to adjust the focal plane 6 of the light pattern 345 emitted from the mirror array 344. By adjusting the focal plane 6 of the light pattern 345 using the objective lens motor 348, and controlling the two-dimensional intensity profile of the light pattern 345 using the mirror position motors 347, the controller 120 is adapted to control the delivery of the light source 110 to the cornea 2 in three dimensions. The three-dimensional pattern is generated by delivering the UV light to selected regions 5 on successive planes (parallel to the focal plane 6), which extend from the corneal surface 2A to the mid-depth region 2B within the corneal stroma. The cross-linking agent 130 introduced into the selected regions 5 is then activated as described above.

By scanning over selected regions 5 of a plane 6 at a particular depth within the cornea 2, the controller 120 can control the activation of the cross-linking agent 130 within the cornea 2 according to a three dimensional profile. In particular, the controller 120 can utilize the laser scanning technology of the laser scanning device 300 to strengthen and stiffen the corneal tissues by activating cross-linking in a three-dimensional pattern within the cornea 2. In an implementation, the objective lens 346 can be replaced by an optical train consisting of mirrors and/or lenses to properly focus the light pattern 345 emitted from the mirror array 344. Additionally, the objective lens motor 348 can be replaced by a motorized device for adjusting the position of the eye 1 relative to the objective lens 346, which can be fixed in space. For example, a chair or lift that makes fine motor step adjustments and adapted to hold a patient during eye treatment can be utilized to adjust the position of the eye 1 relative to the objective lens 346.

Advantageously, the use of laser scanning technologies allows cross-linking to be activated beyond the corneal surface 2A of the cornea 2, at depths where stronger and more stable corneal structure is desired, for example, where structural changes have been generated by an eye therapy treatment. In other words, the application of the initiating element (*i.e*., the light source 110) is applied precisely according to a selected three-dimensional pattern and is not limited to a two-dimensional area at the corneal surface 2A of the cornea 2.

Although the embodiments described herein may initiate cross-linking in the cornea according to an annular pattern defined, for example, by a thermokeratoplasty applicator, the initiation pattern in other embodiments is not limited to a particular shape. Indeed, energy may be applied to the cornea in non-annular patterns, so cross-linking may be initiated in areas of the cornea that correspond to the resulting non-annular changes in corneal structure. Examples of the non-annular shapes by which energy may be applied to the cornea are described in U.S. Patent Serial No. 12/113,672, filed on May 1, 2008.

Some embodiments may employ Digital Micromirror Device (DMD) technology to modulate the application of initiating light, *e*.*g*., UV light, spatially as well as a temporally. Using DMD technology, a controlled light source projects the initiating light in a precise spatial pattern that is created by microscopically small mirrors laid out in a matrix on a semiconductor chip, known as a (DMD). Each mirror represents one or more pixels in the pattern of projected light. The power and duration at which the light is projected is determined as described elsewhere.

Embodiments may also employ aspects of multiphoton excitation microscopy. In particular, rather than delivering a single photon of a particular wavelength to the cornea 2, the delivery system *(e.g.,* 100 in FIG. 1) delivers multiple photons of longer wavelengths, *i*.*e*., lower energy, that combine to initiate the cross-linking. Advantageously, longer wavelengths are scattered within the cornea 2 to a lesser degree than shorter wavelengths, which allows longer wavelengths of light to penetrate the cornea 2 more efficiently than shorter wavelength light. For example, in some embodiments, two photons may be employed, where each photon carries approximately half the energy necessary to excite the molecules in the cross-linking agent 130 that release oxygen radicals. When a cross-linking agent molecule simultaneously absorbs both photons, it absorbs enough energy to release reactive oxygen radicals in the corneal tissue. Embodiments may also utilize lower energy photons such that a cross-linking agent molecule must simultaneously absorb, for example, three, four, or five, photons to release a reactive oxygen radical. The probability of the near-simultaneous absorption of multiple photons is low, so a high flux of excitation photons may be required, and the high flux may be delivered through a femtosecond laser. Because multiple photons are absorbed for activation of the cross-linking agent molecule, the probability for activation increases with intensity. Therefore, more activation occurs where the delivery of light from the light source 110 is tightly focused compared to where it is more diffuse. The light source 110 may deliver a laser beam to the cornea 2. Effectively, activation of the cross-linking agent 330 is restricted to the smaller focal volume where the light source 310 is delivered to the cornea 2 with a high flux. This localization advantageously allows for more precise control over where cross-linking is activated within the cornea 2.

Referring again to FIG. 1, embodiments employing multiphoton excitation microscopy can also optionally employ multiple beams of light simultaneously applied to the cornea 2 by the light source 110. For example, a first and a second beam of light can each be directed from the optical elements 112 to an overlapping region of the cornea 2. The region of intersection of the two beams of light can be a volume in the cornea 2 where cross-linking is desired to occur. Multiple beams of light can be delivered to the cornea 2 using aspects of the optical elements 112 to split a beam of light emitted from the light source 310 and direct the resulting multiple beams of light to an overlapping region of the cornea 2. In addition, embodiments employing multiphoton excitation microscopy can employ multiple light sources, each emitting a beam of light that is directed to the cornea 2, such that the multiple resulting beams of light overlap or intersect in a volume of the cornea 2 where cross-linking is desired to occur. The region of intersection may be, for example, in the mid-depth region 2B of the cornea 2, and may be below the corneal surface 2A. Aspects of the present disclosure employing overlapping beams of light to achieve multi-photon microscopy may provide an additional approach to controlling the activation of the cross-linking agent 130 according to a three-dimensional profile within the cornea 2.

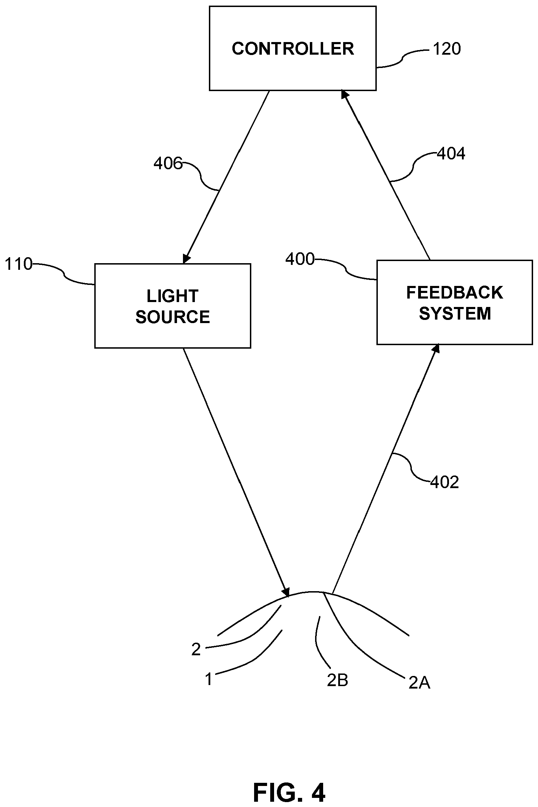

Aspects of the present disclosure, e.g., adjusting the parameters for delivery and activation of the cross-linking agent, can be employed to reduce the amount of time required to achieve the desired cross-linking. In an example implementation, the time can be reduced from minutes to seconds. While some configurations may apply the initiating element (i.e., the light source 110) at a flux dose of 5 J/cm², aspects of the present disclosure allow larger doses of the initiating element, e.g., multiples of 5 J/cm², to be applied to reduce the time required to achieve the desired cross-linking. Highly accelerated cross-linking is particularly possible when using laser scanning technologies (such as in the delivery system 300 provided in FIG. 3) in combination with a feedback system 400 as shown in FIG. 4, such as a rapid video eye-tracking system, described below.

To decrease the treatment time, and advantageously generate stronger cross-linking within the cornea 2, the initiating element (*e.g.,* the light source 110 shown in FIG. 1) may be applied with a power between 30 mW and 1 W. The total dose of energy absorbed in the cornea 2 can be described as an effective dose, which is an amount of energy absorbed through a region of the corneal surface 2A. For example the effective dose for a region of the cornea 2 can be, for example, 5 J/cm², or as high as 20 J/cm² or 30 J/cm². The effective dose delivering the energy flux just described can be delivered from a single application of energy, or from repeated applications of energy. In an example implementation where repeated applications of energy are employed to deliver an effective dose to a region of the cornea 2, each subsequent application of energy can be identical, or can be different according to information provided by the feedback system 400.

Treatment of the cornea 2 by activating cross-linking produces structural changes to the corneal stroma. In general, the optomechanical properties of the cornea changes under stress. Such changes include: straightening out the waviness of the collagen fibrils; slippage and rotation of individual lamellae; and breakdown of aggregated molecular superstructures into smaller units. In such cases, the application of the cross-linking agent 130 introduces sufficient amounts of cross-linking agent to mid-depth regions 2B of the corneal tissue where stronger and more stable structure is desired. The cross-linking agent 130 may be applied directly to corneal tissue that have received an eye therapy treatment and/or in areas around the treated tissue.

To enhance safety and efficacy of the application and the activation of the cross-linking agent, aspects of the present disclosure provide techniques for real time monitoring of the changes to the collagen fibrils with a feedback system 400 shown in FIG. 4. These techniques may be employed to confirm whether appropriate doses of the cross-linking agent 130 have been applied during treatment and/or to determine whether the cross-linking agent 130 has been sufficiently activated by the initiating element (*e.g.,* the light source 110). General studies relating to dosage may also apply these monitoring techniques.

Moreover, real time monitoring with the feedback system 400 may be employed to identify when further application of the initiating element (*e.g.,* the light source 110) yields no additional cross-linking. Where the initiating element is UV light, determining an end point for the application of the initiating element protects the corneal tissue from unnecessary exposure to UV light. Accordingly, the safety of the cross-linking treatment is enhanced. The controller 120 for the cross-linking delivery system can automatically cease further application of UV light when the real time monitoring from the feedback system 400 determines that no additional cross-linking is occurring.

FIG. 4 illustrates a delivery system incorporating the feedback system 400. The feedback system 400 is adapted to gather measurements 402 from the eye 1, and pass feedback information 404 to the controller 120. The measurements 402 can be indicative of the progress of strengthening and stabilizing the corneal tissue. The measurements 402 can also provide position information regarding the location of the eye and can detect movement of the cornea 2, and particularly the regions of the corneal tissue requiring stabilization. The feedback information 404 is based on the measurements 402 and provides input to the controller 120. The controller 120 then analyzes the feedback information 404 to determine how to adjust the application of the initiating element, *e*.*g*., the light source 110, and sends command signals 406 to the light source 110 accordingly. Furthermore, the delivery system 100 shown in FIG. 1 can be adapted to incorporate the feedback system 100 and can adjust any combination of the optical elements 112, the applicator 132, or the light source 110 in order to control the activation of the cross-linking agent 130 within the cornea 2 based on the feedback information 404 received from the feedback system 400.

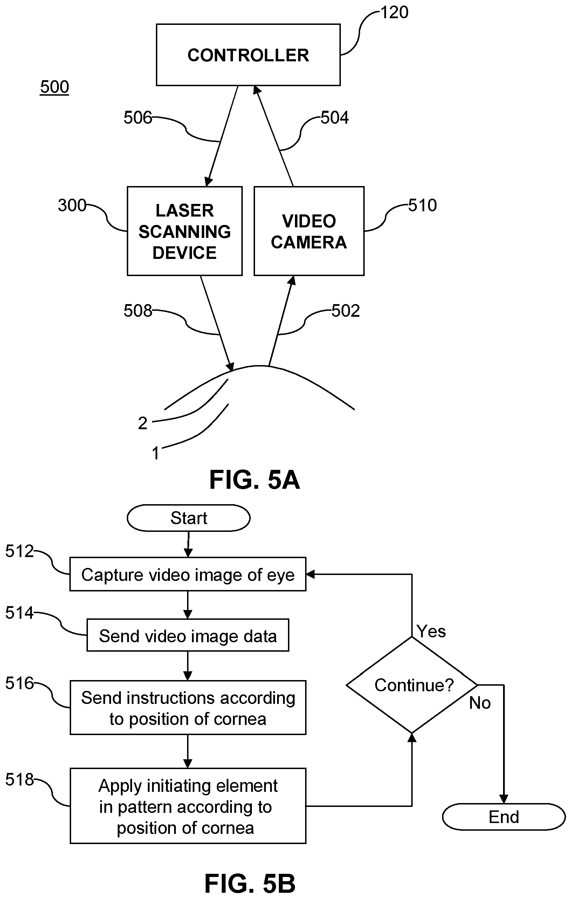

The feedback system 400 can be a video eye-tracking system as shown in FIG. 5A, which illustrates a delivery system 500 for activating cross-linking in the cornea 2 with the laser scanning device 300. The delivery system 500 of FIG. 5A includes a video camera 510 for capturing digital video image data 504 of the eye 1. The video camera 510 generates the video image data 504 of the eye 1 in real time and tracks any movement of the eye 1. The video image data 504 generated by the video camera 510 is indicative of photons 502 reflected from the eye 1. The photons 502 can be reflected from the eye 1 from an ambient light source, or can be reflected from the eye 1 by a light source that is incorporated into the delivery system 500 adapted to direct light to the eye 1 for reflecting back to the video camera 510. Delivery systems including the light source can optionally be adapted with the light source controlled by the controller 120. The delivery system 500 may minimize movement of the eye 1 by minimizing movement of the head, such as, for example, by use of a bite plate described below. However, the eye 1 can still move in the socket, relative to the head.

The real time video image data 504 (*e*.*g*., the series of images captured by the video camera 510) are sent to the controller 120, which may include processing hardware, such as a conventional personal computer or the like. The controller 120 analyzes the data from the video camera 10, for example, according to programmed instructions on computer-readable storage media, e.g., data storage hardware. In particular, the controller 120 identifies the image of the cornea 2 in the video image data 504 and determines the position of the cornea 2 relative to the delivery system 500, and particularly relative to the laser scanning device 300. The controller 120 sends instructions 506 to the laser scanning device 300 to direct a pattern of UV light 508 to the position of the cornea 2. For example, the instructions 506 can adjust optical aspects of the laser scanning device 300 to center the pattern of UV light 508 output from the laser scanning device 300 on the cornea 2. The pattern of UV light 508 activates the cross-linking agent 130 in desired areas and depths of corneal tissue according to aspects of the present disclosure described herein.

In addition, the video image data 504 can optionally include distance information and the controller 130 can be adapted to further analyze the video image data 504 to determine the distance to the cornea 2 from the laser scanning device 508 and can adjust the focal plane of the pattern of UV light 508 directed to the cornea 2. For example, the distance to the cornea 2 may be detected according to an auto-focus scheme that automatically determines the focal plane of the cornea 2, or may be determined according to an active ranging scheme, such as a laser ranging or radar scheme. In an implementation, the video image data 504 can be a series of images, and the controller 120 can be adapted to analyze the images in the series of images individually or in combination to detect, for example, trends in the movement of the cornea 2 in order to predict the location of the cornea 2 at a future time.

FIG. 5B illustrates an exemplary operation of the delivery system 500 shown in FIG. 5A. In step 512, the video camera 510 captures the video image data 504 of the eye 1 based on the photons 502 reflected from the eye 1. In step 514, the video image data 504 is sent to the controller 120. In step 516, the controller 120 sends the instructions 506 to the laser scanning device 300 according to the detected position of the cornea 2. In step 518, the initiating element (e.g., UV light) is applied to the cornea 2 according to the detected position of the cornea 2. Following step 518, a decision is made whether to continue to gather feedback data using the video monitoring system. If feedback data continues to be desired, the exemplary operation returns to step 512 and repeats until it is determined that feedback information is no longer required, at which point the exemplary operation ceases. In an implementation, the delivery system 500 can be adapted to operate according to the steps illustrated in FIG. 5B in real time, and can provide position data about the location of the cornea 2 continuously, or in response to queries from, for example, the controller 120.

In general, the system 500 shown in FIG. 5A can correlate pixels of the video camera 510 with the pixels of the laser scanning device 300, so the real time video image date 504 from the video camera 120 can be employed to direct the pattern of UV light 508 from the laser scanning device 300 accurately to the desired corneal tissue even if there is some movement by the eye 1. The system 500 can be employed to map, associate, and/or correlate pixels in the video camera 510 with pixels in the laser scanning device 300. Advantageously, the system 500 does not require mechanical tracking of the eye 1 and mechanical adjustment (of the laser scanning device 300) to apply the pattern of UV light 508 accurately to the cornea 2.

In sum, implementations of aspects of the present disclosure stabilize a three-dimensional structure of corneal tissue through controlled application and activation of cross-linking in the corneal tissue. For example, the cross-linking agent 130 and/or the initiating element (*e*.*g*., the pattern of UV light 508) are applied in a series of timed and controlled steps to activate cross-linking incrementally. Moreover, the delivery and activation of the cross-linking agent 130 at depths in the cornea 2 depend on the concentration(s) and diffusion times of the cross-linking agent 130 as well as the power(s) and bandwidths of the initiating element. Furthermore, systems may employ laser scanning technologies in combination with a video eye-tracking system to achieve accurate application of the initiating element 222 to the cornea 2.

Another technique for real time monitoring of the cornea 2 during cross-linking treatment employs interferometry with a specialized phasecam interferometer (e.g., manufactured by 4dTechnology, Tucson, AZ). The interferometer takes up to 25 frames per second with a very short exposure so as to substantially minimize motion during an exposure duration. In an example, the exposure time can be less than one millisecond. As the heart beats, the intraocular pressure (IOP) in the eye 1 increases and causes the corneal surface to extend outwardly by a slight amount. The deflection of the cornea 2 is determined by developing a difference map between the peaks and valleys of the cardiac pulsate flow cycles. The deflection of the cornea provides an indicator for the strength of the corneal tissue. The deflection of the cornea 2 may be used to measure changes in the biomechanical strength, rigidity, and/or stiffness during cross-linking treatment. Additionally, comparisons of an amount of deflection observed before and after cross-linking treatment is applied to a cornea 2 may be used to determine a change in biomechanical strength, rigidity, and/or stiffness of the corneal tissue. In general, however, interferometry may be employed to measure corneal strength before and after an eye surgery, before and after any eye treatment, or to monitor disease states. Thus, aspects of the present disclosure employ interferometry as a non-contact technique to determine the surface shape of the cornea 2 and develop a difference map to measure the deflection from IOP. The deflection of the cornea can then be used to determine changes in corneal strength during cross-linking treatment.

To provide control over cross-linking activity, aspects of the present disclosure provide techniques for real time monitoring of the changes in the strength of the corneal tissue. These techniques may be employed to confirm whether appropriate doses of the cross-linking agent have been applied during treatment. Moreover, real time monitoring may be employed to identify when further application of the initiating element yields no additional cross-linking. Where the initiating element is UV light, determining an end point for the application of the initiating element protects the corneal tissue from unnecessary exposure to UV light. Accordingly, the safety of the cross-linking treatment is enhanced. The controller 120 for the cross-linking delivery system (*e*.*g*., the delivery system 100 in FIG. 1) can automatically cease further application of UV light when the real time monitoring determines that no additional cross-linking is occurring.

In addition the video systems and interferometry systems discussed above, still further examples of systems suitable to be included in the feedback system 400 of FIG. 4 include the OCT systems and supersonic shear imaging systems discussed below, which can be operated to provide real-time feedback on the biomechanical properties of the corneal tissue. The information can then be used to develop a treatment plan or dynamically adjust a treatment plan that is suited to the monitored characteristics of the corneal tissue. The treatment plan can be characterized by applications of cross-linking agent, energy doses of initiating element, and selective patterns and/or distributions therefore in order to controllably activate cross-linking in the corneal tissue.

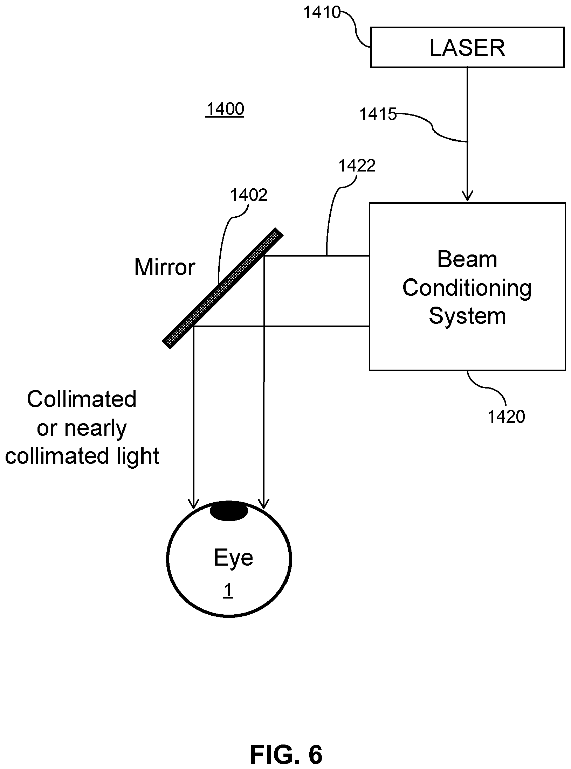

FIG. 6 illustrates an example delivery system 1400 for applying light to an eye 1 incorporating a laser light source 1410 and a beam conditioning system 1420. The laser light source 1400 can emit, for example, ultraviolet or green light suitable for activating a cross-linking agent that is applied to the eye 1. The output of the laser light source 1410 is transferred to the beam conditioning system 1420 via an optical path 1415. The optical path 1415 can include, for example, an optical fiber adapted for delivering the laser light from the laser light source 1410 to the beam conditioning system 1420. The beam conditioning system 1420 receives the output of the laser light source 1410 and emits output light 1422 that is collimated or nearly collimated. In an example implementation of the system 1400, the output light 1422 is reflected by the mirror 1402 and directed to the eye 1. According to an aspect of the system 1400, the intensity of the output light 1422 does not decrease according to the inverse of the square of the distance from the laser light source 1422. By incorporating the laser light source 1422, the system 1400 provides a desired intensity of light to the eye 1 with relatively little sensitivity to the optical distance between the eye 1 and the system 1400. In other words, systems incorporating light sources having intensities that decrease according to an inverse distance squared may require careful alignment of the eye 1 at a particular optical plane to ensure a desired intensity of illumination of the eye 1 is achieved; however, the output light 1422 of the system 1400 provides an intensity that is substantially stable over a range of distances between the eye 1 and the system 1400.

The beam conditioning system 1420 can generally include lenses, mirrors, apertures, and/or other optical elements to condition the beam of light such that the resulting output light 1422 has a non-uniform time-averaged intensity profile. The output light 1422 can activate cross-linking in the eye 1 according to a non-uniform pattern that is related to the non-uniform time-averaged intensity profile. For example, regions of the eye 1 illuminated by portions of the output light 1422 having a relatively greater energy flux can experience more cross linking than regions of the eye 1 illuminated by portions of the output light 1422 having a relatively lesser energy flux.

Aspects of the beam conditioning system 1420 can be similar to the system 300 such that the non-uniform time-averaged intensity profile of the output light 1422 can be generated at least in part by scanning the laser light over an array of selectively alignable mirrors to create a pixelated intensity profile. Additionally or alternatively, the laser light can be diverged or converged and directed to a digital micro mirror device having an array of selectively alignable mirrors to generate a pixelated intensity profile with the digital micro mirror device being imaged to the eye 1.

The laser light may also be passed through one or more fixed or moving apertures to selectively block portions of the beam of light thereby generating the non-uniform time-averaged intensity profile of the output light 1422 imaged on the eye 1. Dynamic adjustments to the non-uniform time-averaged intensity profile can be provided in part by translating and/or rotating apertures adapted to be programmatically positioned to generate the non-uniform time averaged intensity profile. In an implementation, the positions, translations, and/or rotations of the apertures may be carried according to instructions from a controller. Generally, the apertures can be manipulated such that greater amounts of light are blocked, on a time-averaged basis, from regions of the beam corresponding to low-intensity areas of the desired intensity profile, and vice versa. For example, light-blocking portions of the apertures can move relatively more rapidly through regions of the beam corresponding to high-intensity areas of the desired intensity profile and relatively more slowly through regions of the beam corresponding to low-intensity areas of the desired intensity profile. The apertures can include rotating screens having cut-out portions shaped as wedges, shapes similar to a nautilus shell pattern, and/or other shapes. The screens can be rotated in an optical path of the beam of light from the laser 1410. As the cut-out portion of the screen sweeps through regions of the beam of light corresponding to relatively high intensity regions of the non-uniform intensity profile, the angular rotation of the screen can be slowed to a low rate, and while the cut-out portion sweeps through regions of the beam of light corresponding to relatively low intensity regions of the non-uniform time-averaged intensity profile, the angular rotation of the screen can be slowed to a high rate. The same principal can be applied to translating apertures with high rates of motion of a cut-out portion corresponding to low intensity regions of a resulting intensity profile and vice versa.

The beam conditioning system may also have a set of beam steering optics that can scan a converging or diverging beam of light with a specific spot size imaged on the eye 1. The spot intensity distribution, size and shape being modified by the methods described herein.

In implementations, the beam conditioning system 1420 can be programmatically adjusted and controlled by a controller (such as the controller 120 described herein). Generally, similar to aspects described herein in reference to iterative approaches for activating cross-linking, the output light 1422 can be delivered to the eye 1 in one or more doses characterized by a power, bandwidth, duration, and/or intensity profile. Furthermore, aspects of the beam conditioning system 1420 can be automatically adjusted to modify, for example, the overall intensity or power, the non-uniform intensity pattern, the duration, and/or the bandwidth of the output light 1422 for each dose of the output light 1422 delivered to the eye 1. In implementations, the automatic adjustment of each dose of the output light 1422 can be carried out according to feedback information, such as the feedback information provided by the interferometry systems, polarimetry systems, and multi-slit lamp configurations described herein for providing feedback.

Furthermore, ocular coherence tomography (OCT) systems can be employed to provide feedback to the controller (e.g., 120). An OCT system generally utilizes low coherence interferometry of white optical light or near-infrared light interfered with light from a reference surface to characterize regions of interest within a narrow coherence length. OCT systems can employ time domain or frequency domain scanning to generate a high resolution (micrometer scale), three-dimensional (to millimeter depths) profile of the corneal tissue. Examples of OCT systems providing feedback for an eye therapy system are disclosed, for example, in U.S. Provisional Patent Application No. 61/542,269, filed October 2, 2011; and U.S. Provisional Patent Application No. 61/550,576, filed October 24, 2011.

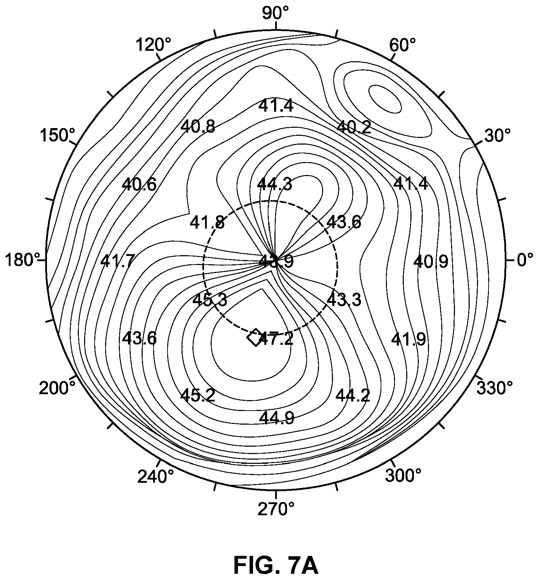

Aspects of the present disclosure also provide systems and methods for treating myopia (i.e., near-sightedness) and/or astigmatism of a patient by activating cross-linking in the patient's corneal tissue. Clinical observations have revealed that myopia can be treated by applying a cross-linking agent (e.g., Riboflavin) to an eye with an applicator, and then activating cross-linking in the corneal tissue of the eye by applying an initiating element, such as UV light. The resulting cross-lining activity in the corneal tissue of the eye has been observed to flatten the shape of the eye, thereby advantageously reducing the corneal power of the cornea so as to correct for myopia. Furthermore, asymmetric flattening of an eye has been observed in patients suffering from astigmatism. In an example clinical treatment, which is discussed next in connection with FIGS. 7A-7C, a patient's astigmatism was observed to be corrected by 0.8 diopters after cross-linking therapy was applied.

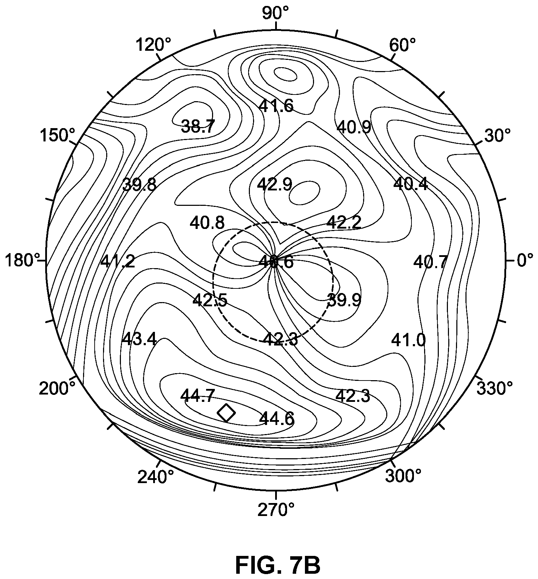

FIG. 7A illustrates an optical power contour map of an eye prior to initiation of cross-linking therapy. The contour map in FIG. 7A illustrates the optical power of the eye measured in diopters with contour lines illustrating regions having uniform optical power. The contour map in FIG. 7A (and FIG. 7B) was produced by an Oculus Pentacam system utilizing rotating Scheimplug cameras to measure corneal thickness and topography (i.e., elevation of the posterior and anterior corneal surface along an axis oriented normal through the center of the eye). Such Pentacam systems are available, for example, from Oculus USA (Lynnwood, WA). The measurements of the elevations of the cornea are converted to axial (Sagittal) radius values within the Pentacam system and the power of the corneal lens is computed based on the axial radius values and based on ray tracing calculations.

As shown in FIG. 7A, the contour map of the eye is characterized by an astigmatism. In particular, a meridian oriented at 159.6 degrees with respect to the horizontal axis is referred to as the flattest meridian (e.g., K1) and has a characteristic optical power of 42.5 diopters. The meridian perpendicular to the flattest meridian (e.g., K2) has a characteristic optical power of 46.1 diopters. Thus, the optical power of the eye is non-uniform about the central optical axis of the eye, and is characterized by a difference of 3.6 diopters between the flattest meridian (K1) and the perpendicular meridian (K2). Thus, the lack of axial uniformity in corneal power about the central point of the corneal contour map shown in FIG. 7A illustrates that the patient suffered from astigmatism.

FIG. 7B illustrates an optical power contour map of the eye shown in FIG. 15A following treatment by cross-linking therapy according to an aspect of the present disclosure. In particular, a cross-linking agent including Riboflavin and benzalkonium chloride (BAC) was applied to the eye. The cross-linking agent was then activated ("initiated") by applying UV light to the cornea in a 3 mm diameter treatment zone approximately centered on the cornea. The UV light was applied in the treatment zone in a dose of 10.8 J/cm² at a rate of 30 mW/cm². In particular, according to an aspect of the present disclosure, the dose of the applied UV light exceeded 5 J/cm². As shown by the contour map in FIG. 7B, the corneal power of the eye was reduced by the cross-linking therapy, thus addressing the patient's myopia observed pre-therapy (FIG. 7A). Furthermore, the astigmatism observed pre-therapy (FIG. 7A) was partially corrected by the cross-linking therapy as well. In particular, in the post-therapy contour map shown in FIG. 7B a meridian oriented at 158.3 degrees with respect to the horizontal axis is referred to as the flattest meridian (e.g., K1) and has a characteristic optical power of 39.8 diopters. The meridian perpendicular to the flattest meridian (e.g., K2) has a characteristic optical power of 42.6 diopters. While the optical power of the eye is non-uniform about the central optical axis of the eye, it is characterized by a difference of only 2.8 diopters between the flattest meridian (K1) and the perpendicular meridian (K2) post-therapy. Thus, the cross-linking therapy improved the patient's pre-therapy astigmatism by 0.8 diopters.

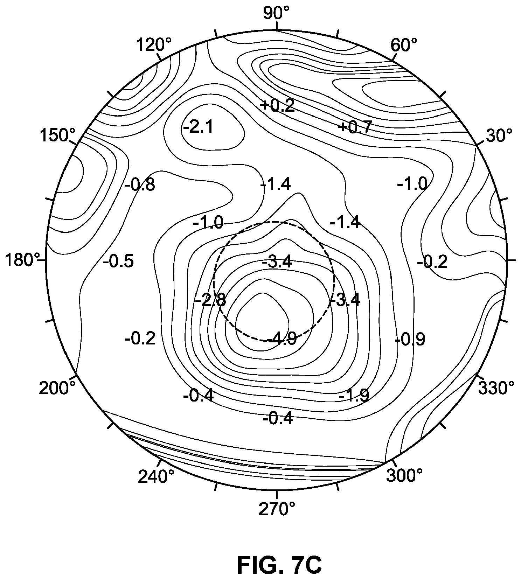

FIG. 7C illustrates a contour map of the difference between the contour map in FIG. 7B and the contour map shown in FIG. 7A. As shown in FIG. 7C, the amount of corneal power adjusted by the cross-linking therapy decreased the corneal power in the treatment zone by approximately 3.4 diopters.

The present disclosure provides techniques for addressing astigmatism that contrast with LASIK techniques for correcting astigmatism. LASIK techniques treat astigmatism by removing corneal tissue from bulging regions of the cornea (i.e., from regions having high corneal power) in order to flatten those regions of the cornea. The removal of corneal tissue from the bulging regions further weakens those regions and undesirably thins those regions of the cornea, making them potentially more susceptible to bulging in the future. In contrast, the cross-linking therapy described herein corrects astigmatism by flattening (and strengthening) bulging regions of the cornea by activating cross-linking therapy in those regions. According to aspects of the present disclosure, corneal thickness and corneal strength is not sacrificed in order to provide optical corrections to the cornea. Aspects of the present disclosure provide for strengthening weakened regions of the cornea (e.g., regions of the cornea that are bulging so as to cause non-uniformities in corneal power) through cross-linking. Furthermore, it has been observed that cross-linking therapy applied to an eye in a uniform treatment zone results in preferential flattening of regions of the treatment zone having relatively greater corneal power (e.g., regions of the cornea with greater axial curvature). This effect of preferential cross-linking activity in higher curvature regions of the corneal tissue results in a partial correction of corneal astigmatism even when cross-linking therapy is initiated according to a uniformly applied pattern within the treatment zone.

While particular clinical results are described in connection with FIGS. 7A through 7C, generally the treatment of the corneal tissue by cross-linking therapy is not limited to flattening corneal tissue to treat myopia and/or astigmatism. Cross-linking therapy can be applied to adjust the optical power of the cornea by selectively flattening and/or strengthening regions of the cornea. It is particularly noted that hyperopia (i.e., "far-sightedness") can be corrected, for example, by activating cross-linking in a ring-shaped region surrounding a central portion of the cornea so as to pinch the cornea and cause the central portion to have an increased optical power, thereby addressing the hyperopia. Furthermore, cross-linking therapy can be applied to an eye principally for the purpose of strengthening the cornea to address corneal thin-ness, weakness, or to reinforce structural changes to the eye applied previously, such as by Photo-Refractive Keratectomy (PRK), LASEK, LASIK, thermokeratoplasty, cataract, scar removal by PRK or Photo-Therapeutic Keratectomy (PTK) or some other form of refractive or ocular surgery. In some examples, cross-linking is activated according to a pattern that corresponds to a region of measured corneal thinness or weakness or according to a pattern that corresponds to a region of expected corneal thinness or weakness based on a previous treatment (e.g., LASIK) to selectively strengthen regions of the corneal tissue known or expected to be relatively weaker than other regions.

According to aspects of the present disclosure, cross-linking therapy treatments applied to an eye can be tuned according to one or more biomechanical properties of the eye, such as the corneal topography (i.e., shape), corneal strength (i.e., stiffness), and/or corneal thickness. Based on the received one or more biomechanical properties, (e.g., corneal thickness), the cross-linking treatment is accordingly adjusted to provide treatment based on the received biomechanical properties. For example, the amount of cross-linking agent and/or dosage of cross-linking activation can be increased for patients having larger corneal thickness. Generally optical correction and/or strengthening of the cornea is applied similar to the descriptions of iterative cross-linking therapy treatments discussed above where the cross-linking agent and/or cross-linking initiating element are each applied in one or more iterations with adjustable characteristics for each iteration. Furthermore, the treatment can be adapted based on feedback information of the biomechanical properties of the cornea that is gathered in real-time during treatment or during breaks in treatment. Generally the developed treatment plan can include a number of applications of the cross-linking agent (e.g., the cross-linking agent 130 shown in FIGS. 1 and 2A), the amount and concentration of the cross-linking agent for each application, the number of treatments of the initiating element (e.g., the initiating element 222 of FIG. 2A), and the timing, duration, power, energy dosage, and pattern of the initiating element for each treatment with the initiating element. As discussed herein, the initiating element can be patterned to apply the initiating element non-uniformly according to a mirror array of digitally controlled micro-mirrors (e.g., FIG. 3 and accompanying description), according to multi-photon excitation microscopy and/or according to the use of masks to selectively block the initiating element.

Additionally and/or alternatively, the non-uniform pattern of the initiating element can also be realized by applying the initiating element to the eye in separate treatment zones with different doses sequentially or continuously applied. For example, one treatment zone can turn off (i.e., ceases to receive the initiating element) while another stays on (i.e., continues to receive the initiating element). The zones can be, for example, annularly shaped about a center point of the eye. There can also be discontinuous zones where no initiating element is applied (e.g., a central zone surrounded by an annulus of no light surrounded by an annulus of light, etc.). The widths of the annular zones ("rings") can be of different dimensions, such as where one annular zone has a width of 1 mm and another has a width of 2 mm. Applying the initiating element in rings on the periphery of the eye without a central spot can result in a hyperopic correction by causing the central region of the eye to have an increased curvature while the periphery is strengthened. Furthermore, the central and surrounding annular treatment zones can be elliptical in shape to correct for astigmatism by preferentially initiating cross-linking in regions of the cornea to correct the astigmatism. Such elliptically shaped annular treatment zones are preferentially oriented with the axis of the annular treatment zones aligned according to the orientation of the astigmatism. The elliptically shaped treatment zones can also be irregularly asymmetric (i.e., having major and minor axis that are not perpendicular and can be situated with distinct center points (centers of mass)). The elliptically shaped treatment zones can also be dictated by the biomechanical properties of the cornea, for example, the corneal topography, the corneal thickness, and/or the corneal strength. These zones may also be defined by the irregular and translating shaped apertures as described herein.

Furthermore, the distribution of the cross-linking agent can be adjusted prior to or during initiation of the cross-linking agent according to the techniques and systems described in commonly assigned U.S. Patent Application No. 13/086,019, filed April 13, 2011.

The one or more biomechanical properties of the eye can be observed pretreatment or can be actively observed during treatment by a feedback system, such as the interferometric feedback system, Oculus Pentacam, 4 slit lamp apparatus, OCT system, and other feedback systems described herein. Additionally and/or alternatively, biomechanical properties of the cornea may be provided according to information from a Supersonic Shear Imaging ("SSI") corneal elasticity measurement system, such as described in, for example, M. Tanter et al., High-Resolution Quantitative Imaging of Cornea Elasticity Using Supersonic Shear Imaging, IEEE Transactions on Medical Imaging, vol. 28, no. 12, Dec. 2009, pp. 1881-1893. Additionally or alternatively, biomechanical properties of the cornea may be provided according to information from an Ocular Response Analyzer for measuring corneal hysteresis in response to a changing optical pressure, available from Reichert, Inc., and as described in Michael Sullivan-Mee, The Role of Ocular Biomechanics In Glaucoma Management, Review of Optometry, Oct. 15, 2008, pp. 49-54, the contents of which is hereby incorporated entirely herein by reference.

The cross-linking agent 122 may be applied to the corneal tissue in an ophthalmic solution, e.g., in the form of eye drops. In some cases, the cross-linking agent 122 is effectively applied to the corneal tissue by removing the overlying epithelium before application. However, in other cases, the cross-linking agent 122 is effectively applied in a solution that transitions across the epithelium into the underlying corneal tissue, *i*.*e*., without removal of the epithelium. For example, a transepithelial solution may combine Riboflavin with approximately 0.1% benzalkonium chloride (BAC) in distilled water. Alternatively, the transepithelial solution may include other salt mixtures, such as a solution containing approximately 0.4% sodium chloride (NaCl) and approximately 0.02% BAC. Additionally, the transepithelial solution may contain methyl cellulose, dextran, or the like to provide a desired viscosity that allows the solution to remain on the eye for a determined soak time.

Although embodiments of the present disclosure may describe stabilizing corneal structure after treatments, such as LASIK surgery and thermokeratoplasty, it is understood that aspects of the present disclosure are applicable in any context where it is advantageous to form a stable three-dimensional structure of corneal tissue through cross-linking. Furthermore, while aspects of the present disclosure are described in connection with the reshaping and/or strengthening of corneal tissue via cross-linking the corneal collagen fibrils, it is specifically noted that the present disclosure is not limited to cross-linking corneal tissue, or even cross-linking of tissue. Aspects of the present disclosure apply generally to the controlled cross-linking of fibrous matter and optionally according to feedback information. The fibrous matter can be collagen fibrils such as found in tissue or can be another organic or inorganic material that is arranged, microscopically, as a plurality of fibrils with the ability to be reshaped by generating cross-links between the fibrils. Similarly, the present disclosure is not limited to a particular type of cross-linking agent or initiating element, and it is understood that suitable cross-linking agents and initiating elements can be selected according to the particular fibrous material being reshaped and/or strengthened by cross-linking.

The present disclosure includes systems having controllers for providing various functionality to process information and determine results based on inputs. Generally, the controllers (such as the controller 120 described throughout the present disclosure) may be implemented as a combination of hardware and software elements. The hardware aspects may include combinations of operatively coupled hardware components including microprocessors, logical circuitry, communication/networking ports, digital filters, memory, or logical circuitry. The controller may be adapted to perform operations specified by a computer-executable code, which may be stored on a computer readable medium.

As described above, the controller 120 may be a programmable processing device, such as an external conventional computer or an on-board field programmable gate array (FPGA) or digital signal processor (DSP), that executes software, or stored instructions. In general, physical processors and/or machines employed by embodiments of the present disclosure for any processing or evaluation may include one or more networked or non-networked general purpose computer systems, microprocessors, field programmable gate arrays (FPGA's), digital signal processors (DSP's), micro-controllers, and the like, programmed according to the teachings of the exemplary embodiments of the present disclosure, as is appreciated by those skilled in the computer and software arts. The physical processors and/or machines may be externally networked with the image capture device(s), or may be integrated to reside within the image capture device. Appropriate software can be readily prepared by programmers of ordinary skill based on the teachings of the exemplary embodiments, as is appreciated by those skilled in the software art. In addition, the devices and subsystems of the exemplary embodiments can be implemented by the preparation of application-specific integrated circuits or by interconnecting an appropriate network of conventional component circuits, as is appreciated by those skilled in the electrical art(s). Thus, the exemplary embodiments are not limited to any specific combination of hardware circuitry and/or software.

Stored on any one or on a combination of computer readable media, the exemplary embodiments of the present disclosure may include software for controlling the devices and subsystems of the exemplary embodiments, for driving the devices and subsystems of the exemplary embodiments, for enabling the devices and subsystems of the exemplary embodiments to interact with a human user, and the like. Such software can include, but is not limited to, device drivers, firmware, operating systems, development tools, applications software, and the like. Such computer readable media further can include the computer program product of an embodiment of the present disclosure for performing all or a portion (if processing is distributed) of the processing performed in implementations. Computer code devices of the exemplary embodiments of the present disclosure can include any suitable interpretable or executable code mechanism, including but not limited to scripts, interpretable programs, dynamic link libraries (DLLs), Java classes and applets, complete executable programs, and the like. Moreover, parts of the processing of the exemplary embodiments of the present disclosure can be distributed for better performance, reliability, cost, and the like.

Common forms of computer-readable media may include, for example, a floppy disk, a flexible disk, hard disk, magnetic tape, any other suitable magnetic medium, a CD-ROM, CDRW, DVD, any other suitable optical medium, punch cards, paper tape, optical mark sheets, any other suitable physical medium with patterns of holes or other optically recognizable indicia, a RAM, a PROM, an EPROM, a FLASH-EPROM, any other suitable memory chip or cartridge, a carrier wave or any other suitable medium from which a computer can read.

While the present disclosure has been described in connection with a number of exemplary embodiments, and implementations, the present disclosure is not so limited, but rather covers various modifications, and equivalent arrangements. In addition, although aspects of the present invention may be described in separate embodiments, it is contemplated that the features from more than one embodiment described herein may be combined into a single embodiment.

## Claims

1. A system for treating an eye, the system comprising:
a feedback system (400) configured to determine at least one biomechanical property of the eye ; and
a delivery system (1400) for directing a dose of light (1422) to the eye to activate a cross-linking agent applied to the eye, the delivery system (1400) including a source (1410) and a beam conditioning system (1420) for the light (1422),
**characterized in that**:
the feedback system (400) is configured to provide real-time feedback information on the determined biomechanical properties of the eye for developing or dynamically adjusting a treatment plan for, at least one of myopia, hyperopia, and astigmatism; and
the beam conditioning system (1420) is configured to modify, according to the feedback information at least one of an intensity, a power, a pattern, a duration, and a bandwidth of the dose of light (1422) directed to the eye, the dose of light (1422) activating the cross-linking agent and generating cross-linking activity that produces a change in optical power that treats the at least one of myopia, hyperopia, and astigmatism .

2. The system according to claim 1, further comprising a controller (120) configured to control the beam conditioning system (1420) according to the feedback information.

3. The system according to any of claims 1-2, wherein the feedback system (400) includes a rotating Scheimpflug system and the at least one biomechanical property includes corneal thickness and topography.

4. The system according to any one of claims 1-2, wherein the feedback system (400) includes an optical coherence tomography (OCT) system.

5. The system according to any one of claims 1-2, wherein the feedback system (400) includes a supersonic shear imaging (SSI) corneal elasticity measurement system.

6. The system according to any one of claims 1-2, wherein the feedback system (400) includes an ocular response analyzer configured to measure corneal hysteresis in response to a changing optical pressure.

7. The system according to any one of claims 1-2, wherein the feedback system (400) includes an interferometry system.

8. The system according to any one of claims 1-2, wherein the feedback system (400) includes a multi-slip lamp system.

9. The system according to any one of claims 1-2, wherein the feedback system (400) includes a polarimetry system.

10. The system according to any of claims 1-9, wherein the feedback system (400) is configured to provide an orientation of astigmatism of the eye, and the beam conditioning system (1420) is configured to modify the dose of light (1422) to be applied in a non-uniform pattern defined by the orientation of the astigmatism.

11. The system according any of claims 1-9, wherein the beam conditioning system (1420) is configured to modify the dose of light (1422) to have a non-uniform time-averaged intensity profile .

12. The system according to any one of claims 1-11, wherein the beam conditioning system (1420) includes at least one of:
a mirror array having a plurality of mirrors arranged in rows and columns, the mirror array being configured to selectively direct the light according to a pixelated intensity pattern, the pixelated intensity pattern having a plurality of pixels corresponding to the plurality of mirrors in the mirror array;
one or more fixed or movable apertures configured to selectively block portions of the beam of light, wherein the beam of light is a laser beam;
and a set of beam steering optics configured to scan the beam of light as a converging or diverging beam of light with a specific spot size imaged on the eye.

13. The system according to any one of claims 1-12, wherein the beam conditioning system (1420) modifies the overall intensity or power, the non-uniform intensity pattern, the duration, and/or the bandwidth of the output light (1422) for each dose of light (1422) to be directed to a selected treatment zone and the cross-linking activity in the treatment zone provides greater flattening in regions of the treatment zone having greater curvature.

## Patentansprüche

1. System zum Behandeln eines Auges, wobei das System Folgendes umfasst:
ein Rückführsystem (400), das konfiguriert ist, um zumindest eine biomechanische Eigenschaft des Auges zu bestimmen; und
ein Zufuhrsystem (1400) zum Richten einer Dosis Licht (1422) auf das Auge, um ein auf das Auge aufgebrachtes Vernetzungsmittel zu aktivieren, wobei das Zufuhrsystem (1400) eine Quelle (1410) und ein Strahlaufbereitungssystem (1420) für das Licht (1422) umfasst,
**dadurch gekennzeichnet, dass**
das Rückführsystem (400) konfiguriert ist, um Echtzeit-Rückführinformationen über die bestimmten biomechanischen Eigenschaften des Auges zum Entwickeln oder dynamischen Einstellen eines Behandlungsplans für zumindest eines aus Myopie, Hyperopie und Astigmatismus bereitzustellen; und
wobei das Strahlaufbereitungssystem (1420) konfiguriert ist, um entsprechend den Rückführinformationen zumindest eines aus einer Intensität, einer Leistung, einem Muster, einer Dauer und einer Bandbreite der auf das Auge gerichteten Dosis Licht (1422) zu verändern, wobei die Dosis Licht (1422) das Vernetzungsmittel aktiviert und Vernetzungsaktivität erzeugt, die eine Veränderung der optischen Leistung hervorruft, welche das zumindest eine aus Myopie, Hyperopie und Astigmatismus behandelt.

2. System nach Anspruch 1, ferner umfassend eine Steuerung (120), die konfiguriert ist, um das Strahlaufbereitungssystem (1420) entsprechend den Rückführinformationen zu steuern.

3. System nach einem der Ansprüche 1 bis 2, wobei das Rückführsystem (400) ein sich drehendes Scheimpflug-System umfasst und die zumindest eine biomechanische Eigenschaft die Hornhautdicke und -topographie umfasst.

4. System nach einem der Ansprüche 1 bis 2, wobei das Rückführsystem (400) ein Optische-Kohärenztomographie-(OCT-)System umfasst.

5. System nach einem der Ansprüche 1 bis 2, wobei das Rückführsystem (400) ein Ultraschall-Scher-Bildgebungs-(SSI-)Hornhautelastizitätsmesssystem umfasst.

6. System nach einem der Ansprüche 1 bis 2, wobei das Rückführsystem (400) ein Augenantwortanalysegerät umfasst, das konfiguriert ist, um als Antwort auf einen sich ändernden optischen Druck Hornhauthysterese zu messen.

7. System nach einem der Ansprüche 1 bis 2, wobei das Rückführsystem (400) ein Interferometriesystem umfasst.

8. System nach einem der Ansprüche 1 bis 2, wobei das Rückführsystem (400) ein Mehrschlitzlampensystem umfasst.

9. System nach einem der Ansprüche 1 bis 2, wobei das Rückführsystem (400) ein Polarimetriesystem umfasst.

10. System nach einem der Ansprüche 1 bis 9, wobei das Rückführsystem (400) konfiguriert ist, um eine Ausrichtung von Astigmatismus des Auges bereitzustellen und wobei das Strahlaufbereitungssystem (1420) konfiguriert ist, um die Dosis Licht (1422) zu verändern, die in einem nicht einheitlichen Muster, welches durch die Ausrichtung des Astigmatismus definiert ist, aufzutragen ist.

11. System nach einem der Ansprüche 1 bis 9, wobei das Strahlenaufbereitungssystem (1420) konfiguriert ist, um die Dosis Licht (1422) zu verändern, so dass es ein nicht einheitliches zeitgemitteltes Intensitätsprofil aufweist.

12. System nach einem der Ansprüche 1 bis 11, wobei das Strahlenaufbereitungssystem (1420) zumindest eines aus Folgenden umfasst:
eine Spiegelanordnung, die eine Vielzahl von Spiegeln aufweist, welche in Zeilen und Spalten angeordnet sind, wobei die Spiegelanordnung konfiguriert ist, um das Licht entsprechend einem gepixelten Intensitätsmuster selektiv zu lenken, wobei das gepixelte Intensitätsmuster eine Vielzahl von Pixeln aufweist, die der Vielzahl von Spiegeln in der Spiegelanordnung entsprechen;
eine oder mehrere festgelegte oder bewegbare Öffnungen, die konfiguriert sind, um selektiv Abschnitte des Lichtstrahls zu blockieren, wobei der Lichtstrahl ein Laserstrahl ist;
und ein Satz von strahllenkenden Optikelementen, der konfiguriert ist, um mit dem Lichtstrahl in Form eines konvergierenden oder divergierenden Lichtstrahls mit einer spezifischen Fleckgröße, die auf dem Auge abgebildet wird, abzurastern.

13. System nach einem der Ansprüche 1 bis 12, wobei das Strahlaufbereitungssystem (1420) für jede Dosis Licht (1422), die auf eine ausgewählte Behandlungszone zu lenken ist, die Gesamtintensität oder -leistung, das nicht einheitliche Intensitätsmuster, die Dauer und/oder die Bandbreite des ausgegebenen Lichts (1422) verändert.

## Revendications

1. Système de traitement d'un oeil, le système comprenant :
un système de rétroaction (400) configuré pour déterminer au moins une propriété biomécanique de l'oeil ; et
un système de distribution (1400) pour diriger une dose de lumière (1422) vers l'oeil afin d'activer un agent de réticulation appliqué à l'oeil, le système de distribution (1400) comprenant une source (1410) et un système de conditionnement de faisceau (1420) pour la lumière (1422),
**caractérisé en ce que** :
le système de rétroaction (400) est configuré pour fournir des informations de rétroaction en temps réel sur les propriétés biomécaniques déterminées de l'oeil afin de développer ou d'ajuster de manière dynamique un plan de traitement pour au moins un parmi une myopie, une hypermétropie et un astigmatisme ; et
le système de conditionnement de faisceau (1420) est configuré pour modifier, en fonction des informations de rétroaction, au moins un parmi une intensité, une puissance, un motif, une durée et une largeur de bande de la dose de lumière (1422) dirigée vers l'oeil, la dose de lumière (1422) activant l'agent de réticulation et générant une activité de réticulation qui produit un changement de puissance optique qui traite au moins un parmi la myopie, l'hypermétropie et l'astigmatisme.

2. Système selon la revendication 1, comprenant en outre un dispositif de commande (120) configuré pour commander le système de conditionnement de faisceau (1420) en fonction des informations de rétroaction.

3. Système selon l'une quelconque des revendications 1 et 2, dans lequel le système de rétroaction (400) comprend un système de Scheimpflug rotatif et la au moins une propriété biomécanique comprend une épaisseur et une topographie de la cornée.

4. Système selon l'une quelconque des revendications 1 à 2, dans lequel le système de rétroaction (400) comprend un système de tomographie par cohérence optique (OCT).

5. Système selon l'une quelconque des revendications 1 à 2, dans lequel le système de rétroaction (400) comprend un système de mesure d'élasticité cornéenne par imagerie par cisaillement supersonique (SSI).

6. Système selon l'une quelconque des revendications 1 à 2, dans lequel le système de rétroaction (400) comprend un analyseur de réponse oculaire configuré pour mesurer une hystérésis de la cornée en réponse à une pression optique changeante.

7. Système selon l'une quelconque des revendications 1 à 2, dans lequel le système de rétroaction (400) comprend un système d'interférométrie.

8. Système selon l'une quelconque des revendications 1 et 2, dans lequel le système de rétroaction (400) comprend un système de lampe à glissement multiple.

9. Système selon l'une quelconque des revendications 1 à 2, dans lequel le système de rétroaction (400) comprend un système de polarimétrie.

10. Système selon l'une quelconque des revendications 1 à 9, dans lequel le système de rétroaction (400) est configuré pour fournir une orientation d'astigmatisme de l'oeil, et le système de conditionnement de faisceau (1420) est configuré pour modifier la dose de lumière (1422) à appliquer selon un motif non uniforme défini par l'orientation de l'astigmatisme.

11. Système selon l'une quelconque des revendications 1 à 9, dans lequel le système de conditionnement de faisceau (1420) est configuré pour modifier la dose de lumière (1422) afin d'avoir un profil d'intensité moyenné dans le temps non uniforme.

12. Système selon l'une quelconque des revendications 1 à 11, dans lequel le système de conditionnement de faisceau (1420) comprend au moins l'un des éléments suivants :
un réseau de miroirs ayant une pluralité de miroirs disposés en rangées et en colonnes, le réseau de miroirs étant configuré pour diriger de manière sélective la lumière selon un motif d'intensité pixelisé, le motif d'intensité pixelisé comportant une pluralité de pixels correspondant à la pluralité de miroirs dans le réseau de miroirs ;
une ou plusieurs ouvertures fixes ou mobiles configurées pour bloquer sélectivement des parties du faisceau de lumière, dans lequel le faisceau de lumière est un faisceau laser ; et un ensemble d'optiques de direction de faisceau configurées pour balayer le faisceau de lumière sous la forme d'un faisceau de lumière convergent ou divergent avec une taille de point spécifique représentée sur l'oeil.

13. Système selon l'une quelconque des revendications 1 à 12, dans lequel le système de conditionnement de faisceau (1420) modifie l'intensité ou la puissance globale, le motif d'intensité non uniforme, la durée et/ou la bande passante de la lumière de sortie (1422) pour chaque dose de lumière (1422) devant être dirigée vers une zone de traitement sélectionnée et l'activité de réticulation dans la zone de traitement produit un plus grand aplatissement dans des régions de la zone de traitement présentant une courbure plus importante.
